# EUROPEAN PATENT SPECIFICATION

(11) **EP 0 781 282 B1**
(45) Date of publication and mention of the grant of the patent: **11.08.1999**
(21) Application number: 95930410.6
(22) Date of filing: 12.09.1995
(51) Int. Cl.: C07D 487/04, A61K 31/495

(54) **[1,2,4]TRIAZOLO[4,3-a]QUINOXALINONE DERIVATIVES, THEIR PREPARATION AND USE**
[1,2,4]-TRIAZOLO[4,3-a]CHINOXALINON-DERIVATE, IHRE HERSTELLUNG UND VERWENDUNG
DERIVES DE LA [1,2,4]TRIAZOLO[4,3-A]QUINOXALINONE, LEUR PREPARATION ET UTILISATION

(30) Priority: 16.09.1994 DK 106694
(43) Date of publication of application: 02.07.1997
(73) Proprietor: NOVO NORDISK A/S, 2880 Bagsvaerd (DK)
(72) Inventor: NIELSEN, Flemming, Elmelund, DK-2830 Virum (DK)
(74) Representative: Mager, Knut
(86) International application number: DK9500365
(87) International publication number: WO9608493

(56) References cited:
- EP-A- 0 040 401
- WO-A-93/06103
- WO-A-93/20077
- WO-A-94/21639
- WO-A-94/26746
- US-A- 5 153 196

## Description

The present invention relates to therapeutically active heterocyclic compounds, a method of preparing the same, pharmaceutical compositions comprising the compounds, and a method of treating therewith.

More specifically, the invention relates to [1,2,4]triazolo[4,3-a]quinoxalinone derivatives, which are useful in the treatment of any indication caused by hyperactivity of excitatory amino acids.

Various related compounds are known from the prior art. WO93/06103 and WO 94/21639 describe similar substituted [1,2,4]triazolo[4,3-a]quinoxalinones having affinity to the AMPA receptors.

Thus, EP-A-0040401 generically describes inter alia triazoloquinoxalin-4-ones substituted at the triazolo ring with e.g. an alkyl, acyl or carbalkoxy group. These compounds are claimed to possess useful anti-hypertensive activity.

In US patent No. 5,153,196 some excitatory amino acid receptor antagonists and methods for the use thereof are disclosed. The compounds conform inter alia to triazoloquinoxalinones having one substituent being H, alkyl, aromatic or CF₃ at the triazolo ring.

Further, international patent publication No. WO 93/20077 deals inter alia with fused quinoxalinone derivatives optionally substituted in the triazolo-ring with lower alkyl which may be substituted by mono- or di(lower alkyl)amino.

L-glutamic acid, L-aspartic acid and a number of other closely related amino acids have in common the ability to activate neurons in the central nervous system (CNS). Biochemical, electrophysiological and pharmacological studies have substantiated this and demonstrated that acidic amino acids are transmitters for the vast majority of excitatory neurons in the mammalian CNS.

Interaction with glutamic acid mediated neurotransmission is considered a useful approach in the treatment of neurological and psychiatric diseases. Thus, known antagonists of excitatory amino acids have shown potent anxiolytic (Stephens et al., Psychopharmacology 90, 143-147, 1985), anticonvulsant (Croucher et al., Science 216 899-901, 1982) and muscle relaxant properties (Turski et al., Neurosci. Lett. 53 321-326, 1985).

It has been suggested that accumulation of extracellular excitatory amino acids, followed by overstimulation of neurons, may explain the neuronal degenerations seen in neurological disorders such as amyotrophic lateral sclerosis, Parkinsonism, Alzheimer's disease, Huntington's disease, epilepsy, and deficiencies of mental and motor performance seen after conditions of brain ischemia, anoxia and hypoglycemia or head and spinal cord trauma (McGeer et al., Nature 263, 517-519, 1976; Simon et al., Science 226, 850-852, 1984; Wieloch, Science 230, 681-683, 1985; Faden et al., Science 244, 798-800, 1989; Turski et al., Nature 349, 414-418, 1991). Other possible indications are psychosis, muscle rigidity, emesis and analgesia.

Excitatory amino acids exert their actions via specific receptors located postsynaptically or presynaptically. Such receptors are at present conveniently subdivided into three groups bases on electrophysiological and neurochemical evidence: 1 the NMDA (N-methyl-D-aspartate) receptors, 2 the AMPA receptors, and 3 the kainate receptors. L-glutamic acid and L-aspartic acid probably activate all the above types of excitatory amino acid receptors and possibly other types as well.

The above mentioned classification of excitatory amino acid receptors into NMDA, AMPA, and kainate receptors is based primarily on the following electrophysiological and neurochemical findings.

1) N-methyl-D-aspartate (NMDA) receptors exhibit high selectivity for the excitant NMDA. Ibotenic acid, L-homocysteic acid, D-glutamic acid and trans-2,3-piperidine dicarboxylic acid (trans-2,3-PDA) exert a strong to moderate agonist activity on these receptors. The most potent and selective antagonists are the D-isomers of the 2-amino-5-phosphonocarboxylic acids, e.g. 2-amino-5-phosphono-valeric acid (D-APV) and 3-[(±)-2-carboxy-piperazin-4-yl]-propyl-1-phosphonic acid (CPP), while moderate antagonist activity is shown by the D-isomers of long chain 2-amino dicarboxylic acids (e.g. D-2-amino-adipic acid) and long chain diaminodicarboxylic acids (e.g. diaminopimelic acid). The NMDA-induced synaptical responses have been extensively investigated in the mammalian CNS, especially in the spinal cord (J. Davies et al., J. Physiol. 297, 621-635, 1979) and the responses have been shown to be strongly inhibited by Mg²⁺.

2) AMPA receptors are activated selectively by AMPA (2-amino-3-hydroxy-5-methyl-4-isoxazolepropionic acid), other potent agonists being quisqualic acid and L-glutamic acid. Glutamic acid diethyl ester (GDEE) is a selective but very weak antagonist of this site. AMPA receptors are relatively insensitive to Mg²⁺.

Glutamate release has long been thought to play a major role in neuronal death resulting from cerebral ischemia (Benveniste, H. et al., J. Neurochem. 43, 1369-1374, 1984). It is well known that NMDA receptor evoked Ca²⁺ influx is an important mechanism in ischemic neuronal cell loss. The non-NMDA receptor coupled ionophor is not permeable to calcium. However, the excitation by the Scaffer collaterals in the CA1 region is excerted by non-NMDA receptors, and this fact is of importance for the events in the postischemic period. Recent studies have shown that selective AMPA antagonists have neuroprotectant effects in global ischemia in the gerbil even when given several hours after reperfusion (Sheardown et al., Science 247, 571-574, 1990).

AMPA antagonists are therefore useful in the treatment of cerebral ischemia.

3) Kainate receptors. Excitatory responses to kainic acid are relatively insensitive to antagonism by NMDA-antagonists and by GDEE, and it has been proposed that kainic acid activates a third subclass of acidic amino acid receptor. Certain lactonized derivatives of kainic acid are selective antagonists (O. Goldberg et al., Neurosci. Lett. 23, 187-191, 1981) and the dipeptide 3-glutamyl-glycine also shows some selectivity for kainate receptors. Ca²⁺ but not Mg²⁺ is a strong inhibitor of kainic acid binding.

The affinity of a substance for one or more of the different types of excitatory amino acid receptors may be studied in simple binding experiments. In essence, the method involves incubation of a particular selected radiolabelled ligand and the particular specific substance to be investigated with brain homogenate which contains the receptor. Measurement of receptor occupancy is made by determination of the radioactivity bound to the homogenate and subtraction of nonspecific binding.

AMPA receptor binding may be studied by using ³H-AMPA as radioligand.

The influence of glutamic acid analogues on secondary effects of glutamate receptor interactions may be studied in vitro by using the phenomenon of spreading depression in chicken retina. Such experiments will provide information as to the efficacies (agonist/antagonist) of the test substances. This is in contrast to binding studies, which only provide information on the affinities of the compounds for the receptor.

It has now been found that the compounds of the invention have affinity for the AMPA receptors and are antagonists in connection with this type of receptor which makes them useful in the treatment of any of the numerous indications caused by hyperactivity of excitatory amino acids, especially neuronal degeneration as are observed in amyotrophic lateral sclerosis, Huntington's chorea, Parkinson's disease, epilepsy and senile dementia or mental and motor dysfunctions seen after conditions of brain ischemia, oxygen deficiency, hypoglycemia and head and spinal cord trauma. Other possible indications ar psychosis, muscle rigidity, emesis, acute and chronic inflammatory disease and analgesia.

The compounds of the invention are represented by the general formula I 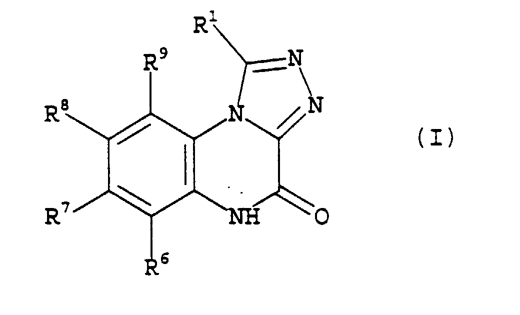 wherein
R¹ is straight C₁₋₆-alkyl substituted with POX'X" and X' and X" independently are hydroxy or C₁₋₆-alkoxy, and
R⁶ and R⁹ are hydrogen;
R⁷ is CF₃;

The compounds of the invention are:
1-(Ethoxy-hydroxy-phosphorylmethyl)-8-(1H-imidazol-1-yl)-7-trifluoromethyl[1,2,4]triazolo[4,3-a]quinoxalin-4(5H)-one;
8-(1 H-lmidazol-1-yl)-1-phosphonomethyl-7-trifluoromethyl[1,2,4]triazolo-[4,3-a]quinoxalin-4(5H)-one;
1 -(Ethoxy-hydroxy-phosphorylmethyl)-8-(2-isopropyl-1H-imidazol-1-yl)-7-trifluoromethyl[1,2,4]triazolo[4,3-a]quinoxalin-4(5H)-one;
8-(2-lsopropyl-1H-imidazol-1-yl)-1-phosphonomethyl-7-trifluoromethyl-[1,2,4]triazolo[4,3-a]quinoxalin-4(5H)-one; and pharmaceutically acceptable salts thereof.

The compounds of the invention may be present in different tautomeric forms. Therefore the invention includes all such tautomeric forms.

Another embodiment of the invention is pharmaceutically acceptable salts of [1,2,4]triazolo[4,3-a]quinoxalinone derivatives. Such salts include those derived from inorganic and organic acids such as hydrochloric acid, hydrobromic acid, acetic acid, sulfuric acid, nitric acid, oxalic acid, fumaric acid, tartaric acid, etc. Other salts include alkali metal salts such as sodium or potassium salts; alkaline earth metal salts, such as calcium or magnesium salts; and ammonium salts.

Further, in another aspect the invention relates to a compound of the invention or a pharmaceutically acceptable salt thereof for use as a medicament, preferably for use as a medicament for treating an indication related to hyperactivity of excitatory neurotransmitters and particularly the AMPA receptors.

The invention also relates to a method of preparing the above mentioned compounds. The present compounds of the invention are prepared by
a) alkylating a compound having the formula II 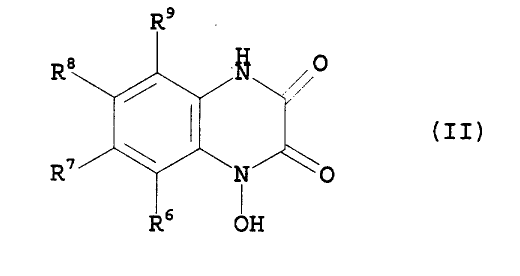 wherein R⁶ and R⁹ are hydrogen, R⁷ is CF₃ and R⁸ is imidazolyl or isopropylimidazolyl with benzylhalogenide to form a compound of the formula III 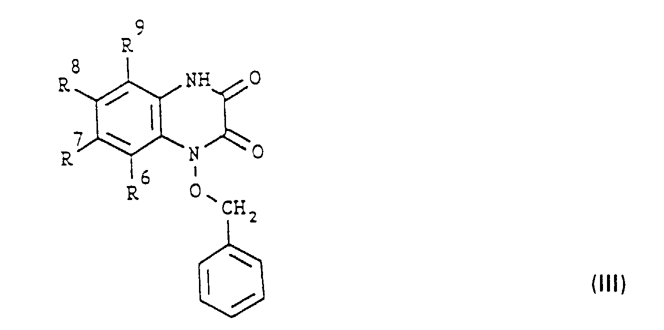 wherein R⁶, R⁷, R⁸ and R⁹ have the meanings defined above, and halogenating the compound to form a compound of the formula IV 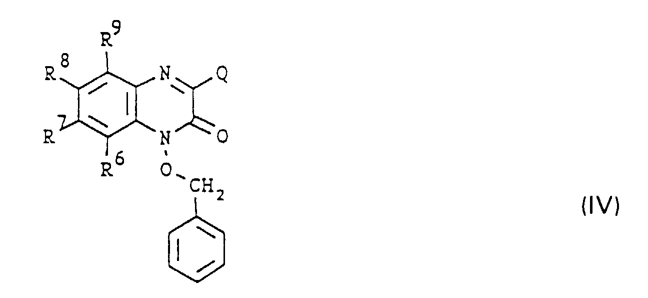 wherein R⁶, R⁷, R⁸ and R⁹ have the meanings defined above and Q is Br, Cl, or I; and reacting the compound with hydrazine to form a compound of the formula V 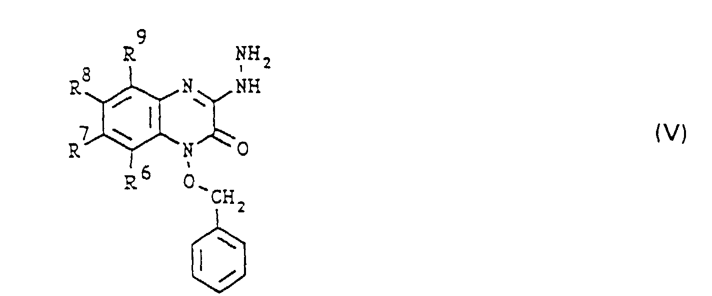 wherein R⁶, R⁷, R⁸ and R⁹ have the meanings defined above, and acylating the compound with an acylchloride with the general formula VI

   R¹-COCl (VI)

   wherein R¹ is ethoxy-hydroxy-phosphorylmethyl or phosphonomethyl to form a compound of the formula VII 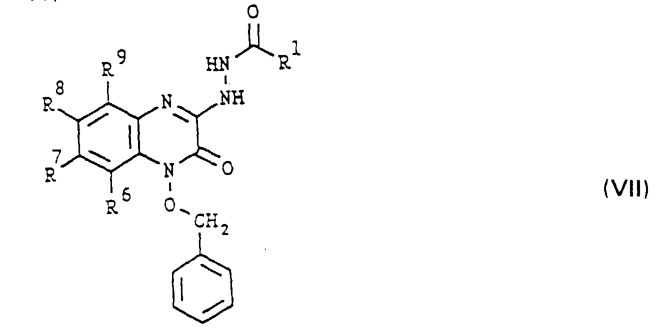 wherein R¹, R⁶, R⁷, R⁸ and R⁹ have the meanings defined above, and hydrogenolysis of the compound to form a compound of the formula VIII 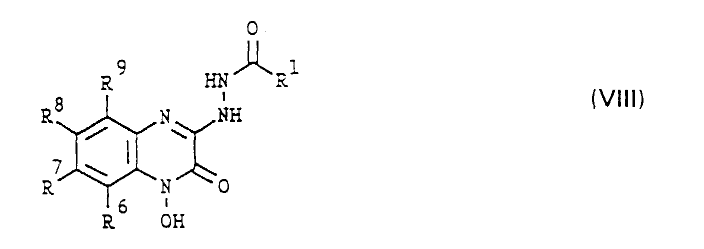 wherein R¹, R⁶, R⁷, R⁸ and R⁹ have the meanings defined above, and followed by thermal cyclization and simultaneous deoxygenation to form a compound of claim 1, or
b) reacting a compound having the formula IX 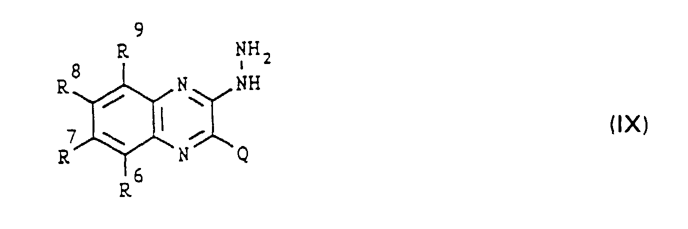 wherein R⁶, R⁷, R⁸ and R⁹ have the meanings defined above, and Q is Br, Cl, or I, with a compound of the general formula VI

   R¹-COCl (VI)

   wherein R¹ has the meaning as defined above to form a compound of the formula Xl 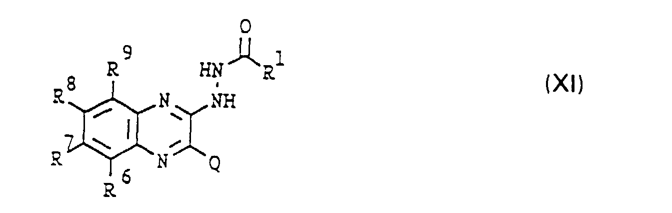 wherein R¹, R⁶, R⁷, R⁸ and R⁹ have the meanings defined above, and Q is Br, Cl, or I, and then either cyclization followed by hydrolysis or simultaneous cyclization and hydrolysis to form a compound of claim 1, or
c) substituting a compound of the formula XII 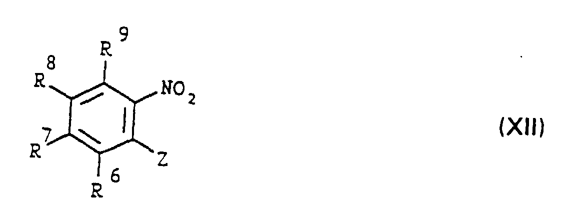 wherein R⁶, R⁷, R⁸ and R⁹ have the meanings defined above and Z is either halogen or C₁₋₆-alkoxy with mono-, di-, or trimethoxy substituted benzylamine to form a compound of formula XIII 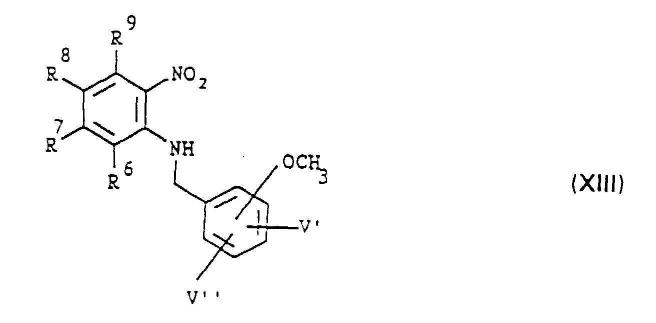 wherein R⁶, R⁷, R⁸ and R⁹ have the meanings defined above, and V' and V" independently are hydrogen or methoxy, and reacting the compound with ethyloxalylchloride to form a compound of formula XIV 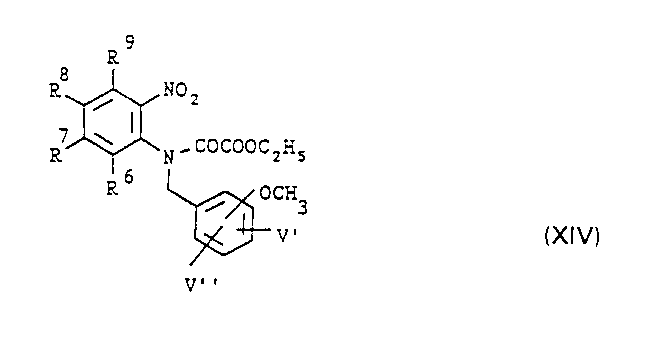 wherein R⁶, R⁷, R⁸ and R⁹ have the meanings defined above, and V' and V" independently are hydrogen or methoxy, and then either hydrogenation to form the intermediate cyclized N-hydroxy compound followed by deoxygenation or cyclization by hydrogenation to form a compound of formula XV 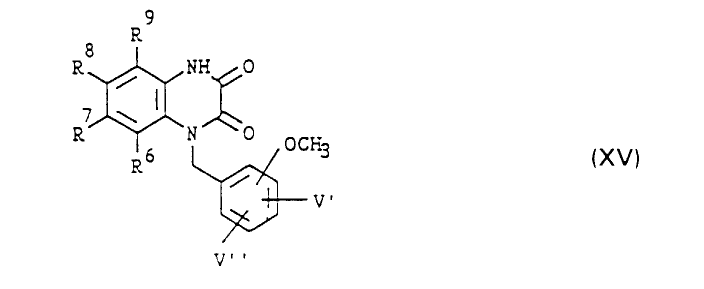 wherein R⁶, R⁷, R⁸ and R⁹ have the meanings defined above, and V' and V" independently are hydrogen or methoxy, halogenating the compound of formula XV, reacting the resulting compound with hydrazine followed by acylating with an acylchloride of the general formula VI as defined above, and then cyclization to form a compound of formula XVI 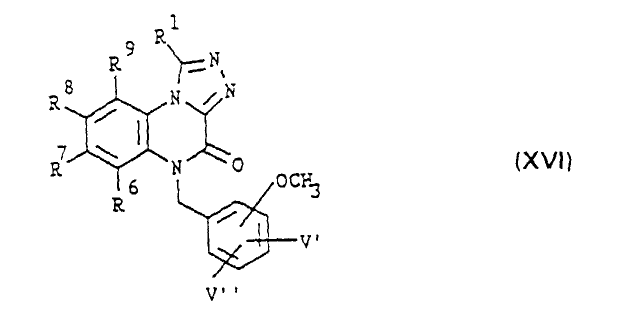 wherein R¹, R⁶, R⁷, R⁸ and R⁹ have the meanings defined above, and V' and V" independently are hydrogen or methoxy, and hydrolysis to form a compound of claim 1, or
d) hydrolysing a compound of claim 1 with aqueous base to form a compound of claim 1, or
e) reacting a compound of claim 1 with halotrimethylsilane to form a compound of claim 1.

Pharmaceutically acceptable salts may be prepared according to standard procedures by treating a compound of formula I with the appropriate acids or bases.

The starting materials for which the preparation is not described herein are either known compounds (e.g. from International appl. no. PCT-DK94/00170) or compounds which may be prepared in analogy with the preparation of known compounds or in analogy with known methods.

The pharmacological properties of the compounds of the present invention can be illustrated by determining their capability for displacing radioactively labelled 2-amino-3-hydroxy-5-methyl-4-isoxazolepropionic acid (AMPA) from the AMPA type receptors. The antagonistic properties of the compounds is demonstrated by their capability to antagonize quisqualic acid stimulated spreading depression in chicken retina.

The displacement activity of the compounds may be shown by determining the IC₅₀ value which represents the concentration (µM) which causes a displacement of 50% of the specific binding of ³H-AMPA.

The antagonism is measured by determining the IC₅₀ value which represents the concentration which produces a 50% maximal inhibition of quisqualic acid stimulated spreading depression in chicken retina.

### ³H-AMPA binding (Test 1)

500 µl of thawed rat cerebral cortical membrane homogenate in Tris-HCI (30 mM), CaCl₂ (2.5 mM) and KSCN (100 mM) pH 7.1 were incubated at 0°C for 30 min. with 25 µl ³H-AMPA (5 nM final concentration) and the test compound and buffer. Nonspecific binding was determined by incubation with L-glutamic acid (600 µM final concentration). The binding reaction was terminated by adding 5 ml of ice-cold buffer followed by filtration through Whatman GF/C glass fibre filters and 2x5 ml wash with ice-cold buffer. Bound radioactivity was measured by scintillation counting. IC₅₀ was determined by Hill analysis of at least four concentrations of test compound.

### Spreading depression (Test 2)

Chicks (3-10 days old) were decapitated, the eyes enucleated and sectioned along the equatorial plane. After removal of the anterior chamber and the vitreous body, the posterior chamber of each eye was placed in a small petri dish containing a physiological saline solution (P.S.S.) of the following composition (mM) NaCI (100), KCI (6.0), CaCl₂ (1.0), MgSO₄ (1.0), NaHCO₃ (30), NaH₂PO₄ (1.0), glucose (20).

The solution was saturated with 100% O₂ and maintained at a temperature of 26°C.

The eyes were initially incubated in normal P.S.S. for 15-30 min. and then transferred to P.S.S. containing quisqualate (1 µg/ml). In this "stimulating solution" S.D.s start spontaneously usually from the edge of the retina, and can be easily observed by eye. The time taken for an S.D. to start in each eye was measured.

After a further 15 min. of incubation in normal P.S.S. the eyes were transferred to normal P.S.S. containing the test compound and incubated for 15 min. Thereafter the eyes were transferred to a "stimulating solution" containing the same concentration of the test compound. The time taken for an S.D. to start in each eye was measured again. The eyes were then placed back in normal P.S.S. and after 15 min. the time taken for S.D. to start was measured again, in order to assess the degree of recovery from any drug effects.

An increase in the time taken for S.D. to start of 30 seconds more than the control time is considered 100% inhibition of S.D. The drug effects therefore are expressed as the percentage maximum response obtained for a given dose. The test value can be quoted therefore as the concentration (µM) of test substance which produces a 50% maximal inhibition (IC₅₀)·

Test results obtained by testing some compounds of the present invention are shown in the following table 1.

**Table 1**

| | TEST 1 | TEST 2 |
|---|---|---|
| Compound of example | IC₅₀ µM | IC₅₀ µM |
| 1 | 0.24 | 1.31 |

The pharmaceutical preparations of compositions comprising the compounds of the invention may be administered to humans or animals by oral, rectal or parenteral route.

An effective amount of the active compound or a pharmaceutically acceptable salt thereof may be determined in accordance with the usual factors, such as the nature and severity of the condition and the weight of the mammal requiring treatment.

Conventional excipients are such pharmaceutically acceptable organic or inorganic carrier substances suitable for parenteral or enteral application which do not deleteriously react with the active compounds.

Examples of such carriers are water, salt solutions, alcohols, polyethylene glycols, polyhydroxyethoxylated castor oil, gelatine, lactose, amylose, magnesium stearate, talc, silicic acid, fatty acid monoglycerides and diglycerides, pentaerythritol fatty acid esters, hydroxymethylcellulose, and polyvinylpyrrolidone.

The pharmaceutical preparations can be sterilized and mixed, if desired, with auxiliary agents, such as lubricants, preservatives, stabilizers, wetting agents, emulsifiers, salt for influencing osmotic pressure, buffers and/or colouring substances and the like, which do not deleteriously react with the active compounds.

Injectable solutions or suspensions, preferably aqueous solutions with the active compound dissolved in polyhydroxylated castor oil, are particularly suitable for parenteral administration.

Ampoules are convenient unit dosage forms.

Tablets, dragees, or capsules containing talc and/or a carrier or binder or the like are particularly suitable for oral administration. The carrier preferably is lactose and/or corn starch and/or potato starch.

A syrup, elixir, or the like can be used in the cases where a sweetened vehicle can be employed or is desired.

Generally, the compounds of this invention are dispensed in unit dosage form comprising 0.5-1000 mg of active ingredient in or together with a pharmaceutically acceptable carrier per unit dosage.

The dosage of the compounds according to this invention is 1-500 mg/day, e.g. about 50-100 mg per dose, when administered to patients, e.g. humans, as a drug.

A typical tablet which may be prepared by conventional tabletting techniques contains:

| Core: | | |
|---|---|---|
| Active compound (as free compound or salt thereof) | | 100 mg |
| Colloidal silicon dioxide (Aerosil^{®}) | | 1.5 mg |
| Cellulose, microcryst. (Avicel^{®}) | | 70 mg |
| Modified cellulose gum (Ac-Di-Sof) | | 7.5 mg |
| Magnesium stearate | | 1 mg |

| Coating: | | |
|---|---|---|
| HPMC | approx. | 9 mg |
| *Mywacett^{®} 9-40T | approx. | 0.9 mg |

| | | |
|---|---|---|
| * Acylated monoglyceride used as plasticizer for film-coating | | |

The free compounds of the present invention which form alkali metal or alkaline earth metal salts may be employed in such salt form. Such alkali metal or earth alkali metal salts are ordinarily formed by reacting the compound with an equivalent amount or excess of the selected alkali metal or earth alkali metal as the hydroxide, frequently and suitably by admixture in the presence of a neutral solvent, from which the salt may be precipitated or recovered in other conventional manner, e.g. by evaporation. Administration of a compound of the invention is often preferably in the form of a pharmaceutically acceptable water-soluble alkali metal or earth alkali metal salt thereof, and orally, rectally, or parenterally in the form of a pharmaceutical composition wherein it is present together with a pharmaceutically acceptable liquid or solid carrier or diluent.

The compounds of the invention, together with a conventional adjuvant, carrier, or diluent, may be placed into the form of pharmaceutical compositions and unit dosages thereof, and in such form may be employed as solids, such as tablets or filled capsules, or liquids, such as solutions, suspensions, emulsions, elixirs, or capsules filled with the same, all for oral use, in the form of suppositories for rectal administration; or in the form of sterile injectable solutions for parenteral (including subcutaneous) use. Such pharmaceutical composition and unit dosage forms thereof may comprise conventional ingredients in conventional proportions, with or without additional active compounds or principles, and such unit dosage forms may contain any suitable effective AMPA antagonistic amount of the active ingredient commensurate with the intended daily dosage range to be employed. Tablets containing 1-500 mg of active ingredient or, more specified 10-200 mg, per tablet, are accordingly suitable representative unit dosage forms.

Due to their high degree of AMPA antagonistic activity and their low toxicity, together presenting a most favourable therapeutic index, the compounds of the invention may be administered to a subject, e.g. a living animal body, in need of such treatment, elimination, alleviation, or amelioration of an indication which is sensitive to a change in the AMPA receptor condition, e.g. sclerosis, Parkinsonism, A!zheimer's disease, Huntington's disease, epilepsy, deficiencies seen after ischemia, anoxia, hypoglycemia, head and spinal cord trauma, psychosis, muscle rigidity, emesis and analgesia, often preferably in the form of an alkali metal or earth alkali metal salt thereof, concurrently, simultaneously, or together with a pharmaceutically acceptable carrier or diluent, especially and preferably in the form of a pharmaceutical composition thereof, whether by oral, rectal, or parenteral (including subcutaneous) route, in an effective amount.

Suitable dosage ranges are 1-500 mg daily, preferably 10-200 mg daily, and especially 50-100 mg daily, depending as usual upon the exact mode of administration, form in which administered, the indication towards which the administration is directed, the subject involved and the body weight of the subject involved, and the preference and experience of the physician or veterinarian in charge.

Such method of treating may be described as the treatment of an indication caused by or related to hyperactivity of the excitatory neurotransmitters, and particularly the AMPA receptors in a subject in need thereof, which comprises the step of administering to the said subject a neurologically effective amount of an AMPA antagonistic compound of the invention, or a pharmaceutically acceptable salt thereof.

Furthermore, the present invention relates to the use of a compound of the invention for preparing a medicament for treating an indication caused by or related to hyperactivity of the excitatory neurotransmitters, and particularly the AMPA receptors in a subject in need thereof.

The invention will now be described in further detail with reference to the following examples:

### EXAMPLE 1

### 1-(Ethoxy-hydroxy-phosphorylmethyl)-8-(1H-imidazol-1-yl)-7-trifluoromethyl[1,2,4]triazolo[4,3-a]quinoxalin-4(5H)-one

### Step a. 1 -Benzyloxy-3-[2-[(diethoxyphosphoryl)acetyl]hydrazino]-6-1H-imidazol-1-yl)-7-trifluoromethylquinoxalin-2(1H)-one

A solution of (diethoxyphosphoryl)acetyl chloride (2.4 g, 11 mmol) in 20 ml of dry tetrahydrofuran was added dropwise to a stirred solution of 1-benzyloxy-3-hydrazino-6-(1H-imidazol--yl)-7-trifluoromethylquinoxalin-2(1H)-one (4.16 g, 10 mmol) and dry triethylamine (1.53 ml, 11 mmol) in 150 ml of dry tetrahydrofuran.

The mixture was stirred for 2 h at room temperature and evaporated to dryness in vacuo. The residue was suspended in 100 ml of water and pH was adjusted to about 7 with saturated aqueous sodium hydrogen carbonate.

The crude product was isolated by filtration and recrystallized from ethylacetate/ether to give 4.7 g (79%) of the title compound. M.p.: 159-161°C.

¹H-NMR IDMSO-d₆): δ 1.23 (t, 6H), 3.04 (d, 2H), 4.06 (quint., 4H), 5.39 (s, 2H), 7.08 (s, 1H), 7.34-7.68 (m, 8H), 7.81 (s, 1H), 10.33 (br. s, 2H, exchangeable).

### Step b. 3-[2-[(Diethoxyphosphoryl)acetyl]hydrazino]-1-hydroxy-6-(1H-imidazol-1-yl)-7-trifluoromethylquinoxalin-2(1H)-one

A suspension of 1-benzyloxy-3-[2-[(diethoxyphosphoryl)acetyl]hydrazino]-6-(1H-imidazol-1-yl)-7-trifluoromethylquinoxalin-2(1H)-one (3.86 g, 6.5 mmol) and 500 mg of 10% palladium on carbon in 250 ml of ethanol was hydrogenated at atmospheric pressure and room temperature for 3 h. The catalyst was removed by filtration and washed with small portions of ethanol. The combined filtrate and washings were evaporated to dryness in vacuo and the residue was triturated with ether to give 2.80 g (86%) of the title compound.

¹H-NMR (DMSO-d₆): δ 1.23 (t, 6H), 3.03 (d, 2H), 4.08 (quint., 4H), 7.07 (s, 1H), 7.40 (s, 1H), 7.41 (s, 1H), 7.83 (s, 1H), 7.90 (s, 1H), 10.1-10.3 (2H), 12.5 (very br. s, 1H).

### Step c. 1 -(Ethoxy-hydroxy-phosphorylmethyl)-8-(1H-imidazol-1-yl)-7-trifluoromethyl[1,2,4]triazolo[4,3-a]quinoxalin-4(5H)-one

A solution of 3-[2-[(diethoxyphosphoryl)acetyl]hydrazinol-1-hydroxy-6-(1H-imidazol-1-yl)-7-trifluoromethylquinoxalin-2(1H)-one (2.52 g, 5 mmol) and triphenylphosphine (2.62 g, 10 mmol) in 100 ml of glacial acetic acid was stirred at 120°C for 23 h. To the cooled mixture was added 150 ml of ether.

The precipitated solid was isolated by filtration and washed with ether and acetone to give 1.09 g (43%) of the title compound. M.p. 324-328°C.

¹H-NMR (DMSO-d₆): δ 1.10 (t, 3H), 1.91 (s, 3H), 3.88 (quint., 2H), 4.05 (d, 2H), 7.21 (s, 1H), 7.50 (s, 1H), 7.82 (s, 1H), 8.06 (s, 1H), 8.50 (s, 1H), 12.49 (s, 1H).

### EXAMPLE 2

### 8-(1H-lmidazol-1-yl)-1-phosphonomethyl-7-trifluoromethyl[1,2,4]triazolo-[4,3-a]quinoxalin-4(5H)-one

Bromotrimethylsilane (2 ml, 14 mmol) was added dropwise to a stirred solution of 1 -(ethoxy-hydroxy-phosphorylmethyl)-8-(1H-imidazol-1-yl)-7-trifluoromethyl[1,2,4]triazolo[4,3-a]quinoxalin-4(5H)-one (450 mg) in 20 ml of dry N,N-dimethylformamide. The solution was stirred at room temperature for 3 days, added 25 ml of water and evaporated to dryness in vacuo. The oily residue was triturated with a small amount of water and the resulting precipitate was isolated by filtration and washed with small portions of cold water, ethanol and ether to give 210 mg of the title compound. M.p. 349-350°C.

¹H-NMR (DMSO-d₆): δ 3.98 (d, 2H), 7.20 (s, 1H), 7.48 (s, 1H), 7.83 (s, 1H), 8.05 (s, 1H), 8.50 (s, 1H), 12.4 (br. s, 1H).

| | | | | | | |
|---|---|---|---|---|---|---|
| (C₁₄H₁₀N₆F₃O₄P·1.5H₂O) | | | | | | |
| Calc. | C | 38.11, | H | 2.97, | N | 19.05 |
| Found | C | 38.21, | H | 3.02, | N | 18.75 |

### EXAMPLE 3

### 1-(Ethoxy-hydroxy-phosphorylmethyl)-8-(2-isopropyl-1H-imidazol-1-yl)-7-trifluoromethyl[1,2,4]triazolo[4,3-a]quinoxalin-4(5H)-one

### Step a. 1 -Benzyloxy-3-chloro-6-(2-isopropyl-1H-imidazol-1-yl)-7-trifluoromethylquinoxalin-2(1H)-one

A solution of 20% phosgene in toluene (18 ml, 34.7 mmol) was added dropwise to a stirred solution of 5.04 g (11.3 mmol) 1-benzyloxy-6-(2-isopropyl-1H-imidazol-1-yl)-7-trifluoromethylquinoxaline-2,3(1H,4H)-dione in 50 ml of dry N,N-dimethylformamide at 0°C. The mixture was stirred at 25°C overnight and the precipitated solid was isolated by filtration and washed with ether to give 4.81 g (85%) of the title compound as the hydrochloride.

¹H-NMR (DMSO-d₆): δ 1.33 (distorted t, 6H), 2.65-2.80 (m, 1H), 5.87 (s, 2H), 7.40-7.67 (m, 5H), 7.77 (s, 1H), 7.92 (s, 1H), 7.96 (s, 1H), 8.62 (s, 1H).

### Step b. 1-Benzyloxy-3-hydrazino-6-(2-isopropyl-1H-imidazol-1-yl)-7-trifluoromethylquinoxalin-2(1H)-one

A mixture of 1-benzyloxy-3-chloro-6-(2-isopropyl-1H-imidazol-1-yl)-7-trifluoromethylquinoxalin-2(1H)-one hydrochloride (4.8 g, 9.6 mmol) and hydrazine hydrate (2.0 ml, 41 mmol) in 100 ml of dichloromethane was stirred at 0°C for 2 h. The mixture was evaporated to dryness in vacuo and the residue was triturated with water to give 4.1 g (93%) of the title compound.

¹H-NMR (DMSO-d₆): δ 1.10 (d, 6H), 2.42-2.65 (m, 1H), 5.32 (s, 2H), 6.91 (s, 1H), 7.07 (s, 1H), 7.28 (s, 1H), 7.37-7.47 (m, 3H), 7.50 (s, 1H), 7.52-7.60 (m, 2H).

### Step c. 1-Benzyloxy-3-[2-[(diethoxyphosphoryl)acetyl]hydrazino]-6-(2-isopropyl-1H-imidazol-1-yl)-7-trifluoromethylquinoxalin-2(H)-one

A solution of (diethoxyphosphoryl)acetyl chloride (1.93 g, 9 mmol) in 25 ml of dry tetrahydrofuran was added dropwise to a stirred solution of 1-benzyloxy-3-hydrazino-6-(2-isopropyl-1H-imidazol-1-yl)-7-trifluoromethylquinoxalin-2(1H)-one (4.0 g, 8.7 mmol) and dry triethylamine (1.25 ml, 9 mmol) in 75 ml of dry tetrahydrofuran.

The mixture was stirred overnight at room temperature and evaporated to dryness. The residue was taken up in 500 ml of water and filtered. The filtrate was adjusted to about pH 8 with saturated aqueous sodium hydrogen carbonate and extracted with ethyl acetate (5 x 50 ml). The combined organic extract was dried (anhydrous sodium sulfate), filtered and evaporated to dryness in vacuo to give 4.3 g (78%) of the crude title compound.

¹H-NMR (DMSO-d₆); δ 1.25 (t, 12H), 2.63-2.77 (m, 1H), 3.03 (d, 2H), 4.07 (quint., 4H), 5.40 (s, 2H), 7.38-7.48 (m, 3H), 7.55-7.63 (m, 2H), 7.68 (s, 1H), 7.79 (s, 2H), 7.87 (s, 1H), 10.4 (br. s, 1H), 10.5 (br. s, 1H).

### Step d. 3-[2-[(Diethoxyphosphoryl)acetyl]hydrazino]-1-hydroxy-6-(2-isopropyl-1H-imidazol-1-yl)-7-trifluoromethylquinoxalin-2(1H)-one

A suspension of 1-benzyloxy-3-[2-[(diethoxyphosphoryl)acetyl]hydrazino]-6-(2-isopropyl-1H-imidazol-1-yl)-7-trifluoromethylquinoxalin-2(1H)-one (4.3 g, 6.6 mmol) and 100 mg of 5% palladium on carbon in 100 ml of ethanol was hydrogenated for 2 h. The catalyst was removed by filtration and washed with ethanol. The combined filtrate was evaporated to dryness in vacuo and the residue was triturated with ether to give 3.0 g (83%) of the title compound. M.p. > 190°C decomp.

¹H-NMR (DMSO-d₆): δ ≈ 1.09 (two d, 6H), 1.22 (t, 6H), 2.46-2.69 (m, 1H), 3.02 (d, 2H), 4.05 (quint. 4H), 6.94 (s, 1H), 7.15 (s, 1H), 7.38 (s, 1H), 7.94 (s, 1H).

### Step e. 1-(Ethoxy-hydroxy-phosphorylmethyl)-8-(2-isopropyl-1H-imidazol-1-yl)-7-trifluoromethyl[1,2,4]triazolo[4,3-a]quinoxalin-4(5H)-one

A solution of 3-[2-[(diethoxyphosphoryl)acetyl]hydrazino]-1-hydroxy-6-(2-isopropyl-1H-imidazol-1-yl)-7-trifluoromethylquinoxalin-2(1H)-one (3.0 g, 5.5 mmol) and triphenylphosphine (2.9 g, 11 mmol) in 50 ml of glacial acetic acid was stirred at 120°C for 23 h. The mixture was allowed to cool to room temperature and the precipitated solid was isolated by filtration and washed with ether to give 1.84 g of the title compound. M.p. 335-338°C.

¹H-NMR (CF₃COOD): δ 1.68 (t, 3H), 1.82 (distorted t, 6H), 3.37-3.57 (m, 1H), 4.62 (quint. 2H), 4.78-5.00 (m, 2H), 7.88 (s, 2H), 8.69 (s, 1H), 9.35 (s, 1H).

### EXAMPLE 4

### 8-(2-lsopropyl-1H-imidazol-1-yl)-1-phosphonomethyl-7-trifluoromethyl-[1,2,4]triazolo[4,3-a]quinoxalin-4(5H)-one

Bromotrimethylsilane (2.5 ml, 17.5 mmol) was added dropwise to a stirred solution of 1-(ethoxy-hydroxy-phosphorylmethyl)-8-(2-isopropyl-1H-imidazol-1-yl)-7-trifluoromethyl[1,2,4]triazolo[4,3-a]quinoxalin-4(5H)-one (1.5 g, 2.7 mmol) in 10 ml of dry N,N-dimethylformamide.

The solution was stirred at room temperature for 3 days and evaporated to dryness in vacuo. The oily residue was triturated with 20 ml of water, the resulting precipitated solid was isolated by filtration and washed with water. The crude product was treated with 50 ml of 1M potassium dihydrogen phosphate buffer (pH 7.4) and the resulting potassium salt was filtered off, then dissolved in 50 ml of water, treated with decolorising charcoal, filtered and finally reprecipitated with conc. hydrochloric acid. The product was isolated by filtration, washed with water and dried to give 0.76 g (54%) of the title compound. M.p. >325°C.

¹H-NMR (DMSO-d₆): δ 1.07 (d, 3H), 1.19 (d, 3H), 2.79 (quint., 1H), 3.57-4.01 (m, 2H), 7.04 (s, 1H), 7.17 (s, 1H), 7.88 (s, 1H), 8.48 (s, 1H).

## Claims

1. A compound which is
1-(Ethoxy-hydroxy-phosphorylmethyl)-8-(1H-imidazol-1-yl)-7-trifluoromethyl[1,2,4]triazolo[4,3-a]quinoxalin-4(5H)-one;
8-(1H-lmidazol-1-yl)-1-phosphonomethyl-7-trifluoromethyl[1,2,4]triazolo-[4,3-a]quinoxalin-4(5H)-one;
1-(Ethoxy-hydroxy-phosphorylmethyl)-8-(2-isopropyl-1H-imidazol-1-yl)-7-trifluoromethyl[1,2,4]triazolo[4,3-a]quinoxalin-4(5H)-one;
8-(2-lsopropyl-1H-imidazol-1-yl)-1-phosphonomethyl-7-trifluoromethyl[1,2,4]triazolo[4,3-a]quinoxalin-4(5H)-one.
and pharmaceutically acceptable salts thereof.

2. A pharmaceutical composition comprising as active component a compound according to claim 1 or a pharmaceutically acceptable salt thereof and a pharmaceutically acceptable carrier or diluent.

3. A pharmaceutical composition according to claim 2 in the form of a dosage unit containing about 10-200 mg of the active compound.

4. The use of a compound according to claim 1 or a pharmaceutically acceptable salt thereof as a medicament.

5. The use of a compound according to claim 1 or a pharmaceutically acceptable salt thereof for preparing a medicament for treating an indication related to hyperactivity of excitatory neurotransmitters.

6. A pharmaceutical composition suitable for use in the treatment of an indication related to hyperactivity of the excitatory neurotransmitters, which comprises as active component a compound according to claim 1 or a pharmaceutically acceptable salt thereof and a pharmaceutically acceptable carrier or diluent.

7. Use of a compound according to claim 1 for treating an indication related to hyperactivity of the excitatory neurotransmitters in a subject in need thereof, which comprises the step of administering to the said subject a neurologically effective AMPA antagonistic amount of a compound according to claim 1 or a pharmaceutically acceptable salt thereof.

8. Use according to claim 7, wherein the indication is related to cerebral ischemia.

9. A method of preparing the compounds of claim I, which comprises
a) alkylating a compound having the formula II 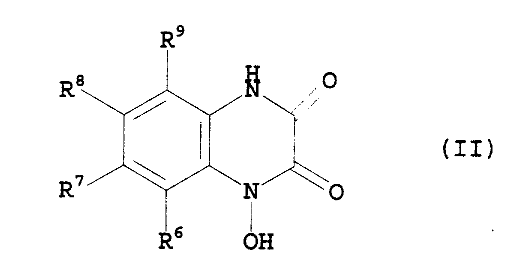 wherein R⁶ and R⁹ are hydrogen, R⁷ is CF₃ and R⁸ is imidazolyl or isopropylimidazolyl with benzylhalogenide to form a compound of the formula III 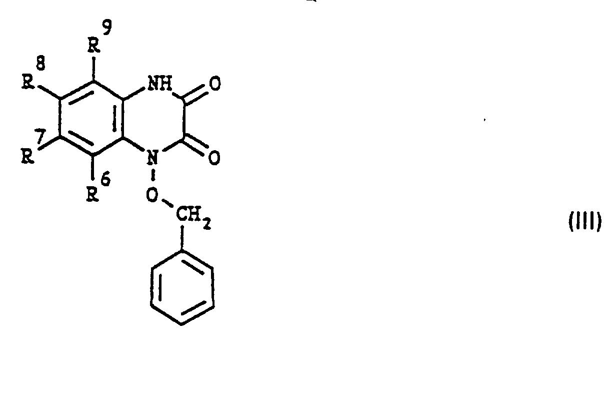 wherein R⁶, R⁷, R⁸ and R⁹ have the meanings defined above, and halogenating the compound to form a compound of the formula IV 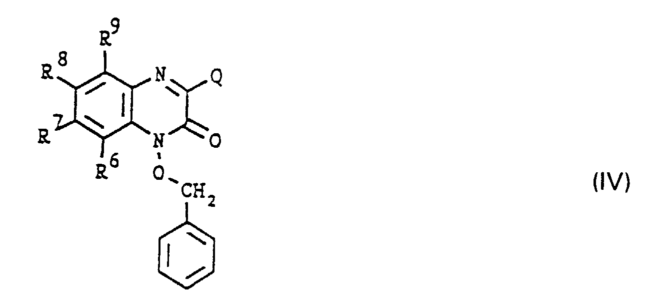 wherein R⁶, R⁷, R⁸ and R⁹ have the meanings defined above and Q is Br, Cl or I; and reacting the compound with hydrazine to form a compound of the formula V 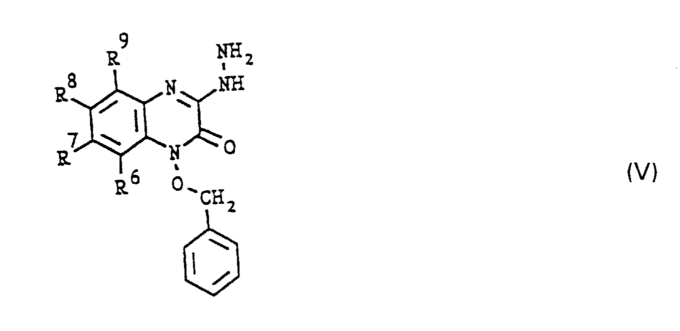 wherein R⁶, R⁷, R⁸ and R⁹ have the meanings defined above, and acylating the compound with an acylchloride with the general formula VI
R¹-COCl (VI)
wherein R¹ is ethoxy-hydroxy-phosphorylmethyl or phosphonomethyl to form a compound of the formula VII 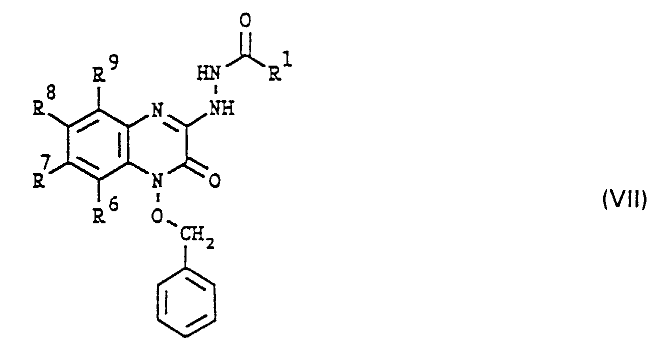 wherein R¹, R⁶, R⁷, R⁸ and R⁹ have the meanings defined above, and hydrogenolysis of the compound to form a compound of the formula VIII 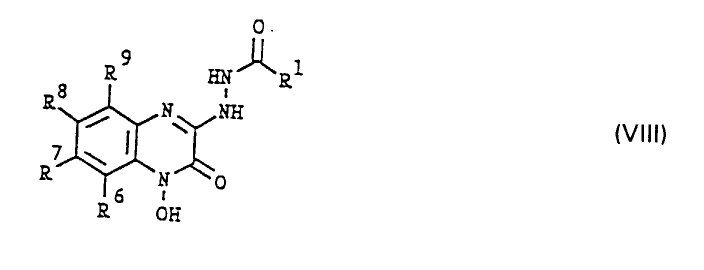 wherein R¹, R⁶, R⁷, R⁸ and R⁹ have the meanings defined above, and followed by thermal cyclization and simultaneous deoxygenation to form a compound of claim 1, or
b) reacting a compound having the formula IX 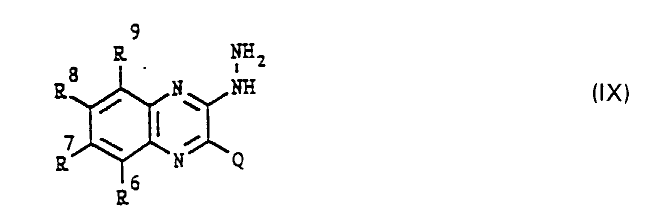 wherein R⁶, R⁷, R⁸ and R⁹ have the meanings defined above, and Q is Br, Cl or I, with a compound of the general formula VI
R¹-COCl (VI)
wherein R¹ has the meaning as defined above to form a compound of the formula Xl 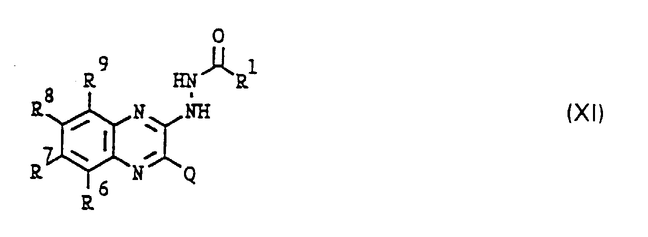 wherein R¹, R⁶, R⁷, R⁸ and R⁹ have the meanings defined above, and Q is Br, Cl, or I, and then either cyclization followed by hydrolysis or simultaneous cyclization and hydrolysis to form a compound of claim 1, or
c) substituting a compound of the formula XII 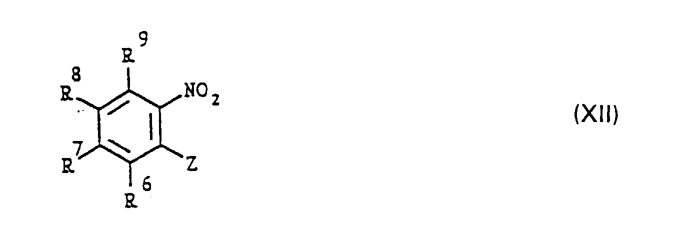 wherein R⁶, R⁷, R⁸ and R⁹ have the meanings defined above and Z is either halogen or C₁₋₆-alkoxy with mono-, di-, or trimethoxy substituted benzylamine to form a compound of formula XIII 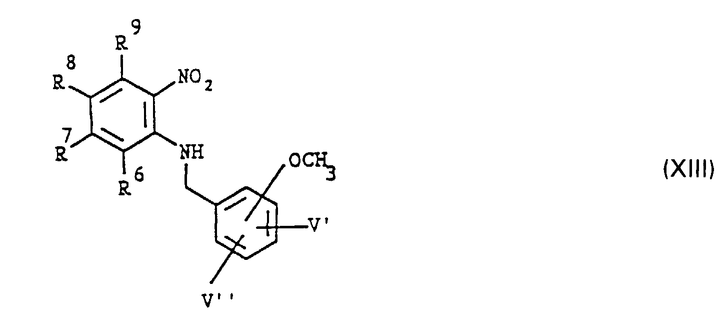 wherein R⁶, R⁷, R⁸ and R⁹ have the meanings defined above, and V' and V" independently are hydrogen or methoxy, and reacting the compound with ethyloxalylchloride to form a compound of formula XIV 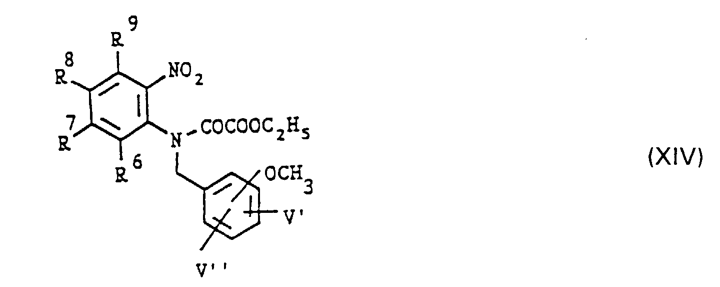 wherein R⁶, R⁷, R⁸ and R⁹ have the meanings defined above, and V' and V" independently are hydrogen or methoxy, and then either hydrogenation to form the intermediate cyclized N-hydroxy compound followed by deoxygenation or cyclization by hydrogenation to form a compound of formula XV 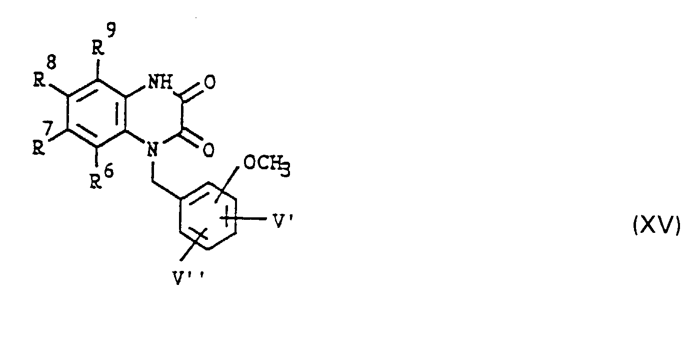 wherein R⁶, R⁷, R⁸ and R⁹ have the meanings defined above, and V' and V" independently are hydrogen or methoxy, halogenating the compound of formula XV, reacting the resulting compound with hydrazine followed by acylating with an acylchloride of the general formula VI as defined above, and then cyclization to form a compound of formula XVI 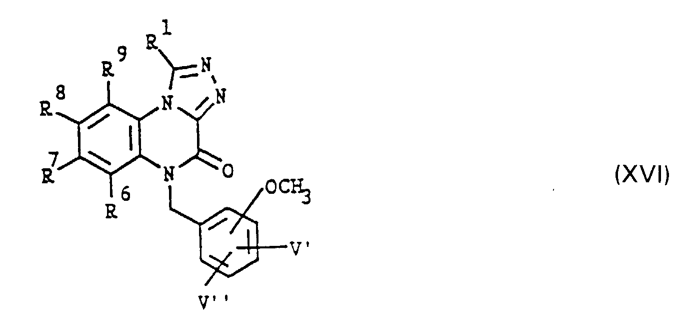 wherein R¹, R⁶, R⁷, R⁸ and R⁹ have the meanings defined above, and V' and V" independently are hydrogen or methoxy, and hydrolysis to form a compound of claim 1, or
d) hydrolysing a compound of claim 1 with aqueous base to form a compound of claim 1, or
e) reacting a compound of claim 1 with halotrimethylsilane to form a compound of claim 1.

10. A process for the manufacture of a pharmaceutical composition to be used in the treatment of an indication related to hyperactivity of the excitatory neurotransmitters which process comprises bringing a compound of claim 1 or a pharmaceutically acceptable salt thereof into a galenical dosage form.

## Patentansprüche

1. Verbindung, bei der es sich um
1-(Ethoxy-hydroxy-phosphorylmethyl)-8-(1H-imidazol-1-yl)-7-trifluormethyl[1,2,4]triazolo[4,3-a]chinoxalin-4(5H)-on;
8-(1H-Imidazol-1-yl)-1-phosphonomethyl-7-trifluormethyl[1,2,4]triazolo[4,3-a]chinoxalin-4(5H)-on;
1-(Ethoxy-hydroxy-phosphorylmethyl)-8-(2-isopropyl-1H-imidazol-1-yl)-7-trifluormethyl[1,2,4]triazolo[4,3-a]chinoxalin-4(5H)-on;
8-(2-Isopropyl-1H-imidazol-1-yl)-1-phosphonomethyl-7-trifluormethyl[1,2,4] triazolo[4,3-a]chinoxalin-4(5H)-on
und pharmazeutisch unbedenkliche Salze davon handelt.

2. Pharmazeutische Zusammensetzung, die als Wirkstoff eine Verbindung nach Anspruch 1 oder ein pharmazeutisch unbedenkliches Salz davon und einen pharmazeutisch unbedenklichen Träger oder ein pharmazeutisch unbedenkliches Verdünnungsmittel enthält.

3. Pharmazeutische Zusammensetzung nach Anspruch 2 in Form einer Dosierungseinheit, die etwa 10 bis 200 mg des Wirkstoffs enthält.

4. Verwendung einer Verbindung nach Anspruch 1 oder eines pharmazeutisch unbedenklichen Salzes davon als Arzneimittel.

5. Verwendung einer Verbindung nach Anspruch 1 oder eines pharmazeutisch unbedenklichen Salzes davon zur Herstellung eines Arzneimittels zur Behandlung einer mit Hyperaktivität von exzitatorischen Neurotransmittern verbundenen Indikation.

6. Zur Verwendung bei der Behandlung einer mit Hyperaktivität der exzitatorischen Neurotransmitter verbundenen Indikation geeignete pharmazeutische Zusammensetzung, die als Wirkstoff eine Verbindung nach Anspruch 1 oder ein pharmazeutisch unbedenkliches Salz davon und einen pharmazeutisch unbedenklichen Träger oder ein pharmazeutisch unbedenkliches Verdünnungsmittel enthält.

7. Verwendung einer Verbindung nach Anspruch 1 zur Behandlung einer mit Hyperaktivität der exzitatorischen Neurotransmitter verbundenen Indikation, bei dem man man einem Patienten, der einer derartigen Behandlung bedarf, eine neurologisch wirksame AMPA-agonistische Menge einer Verbindung nach Anspruch 1 oder eines pharmazeutisch unbedenklichen Salzes davon verabreicht.

8. Verwendung nach Anspruch 7, bei der die Indikation mit cerebraler Ischämie verbunden ist.

9. Verfahren zur Herstellung der Verbindungen der Formel I, bei dem man
a) eine Verbindung der Formel II 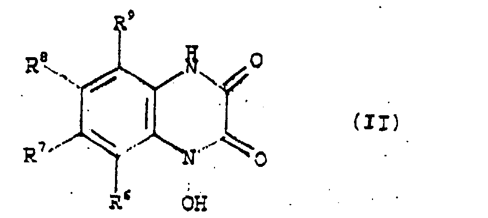 worin R⁶ und R⁹ für Wasserstoff stehen, R⁷ für CF₃ und R⁸ für Imidazolyl oder Isopropylimidazolyl steht, mit Benzylhalogenid zu einer Verbindung der Formel III 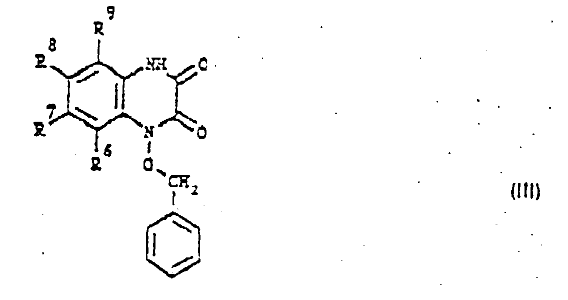 worin R⁶, R⁷, R⁸ und R⁹ die oben angegebenen Bedeutungen haben, alkyliert, die Verbindung zu einer Verbindung der Formel IV 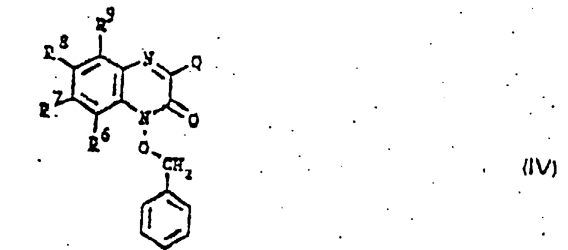 worin R⁶, R⁷, R⁸ und R⁹ die oben angegebenen Bedeutungen haben und Q für Br, Cl oder I steht, halogeniert, die Verbindung mit Hydrazin zu einer Verbindung der Formel V 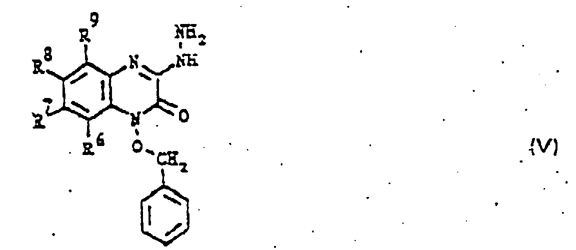 worin R⁶, R⁷, R⁸ und R⁹ die oben angegebenen Bedeutungen haben, umsetzt, die Verbindung mit einem Acylchlorid der allgemeinen Formel VI
R¹-COCl (VI)
worin R¹ für Ethoxy-hydroxy-phosphorylmethyl oder Phosphonomethyl steht, zu einer Verbindung der Formel VII 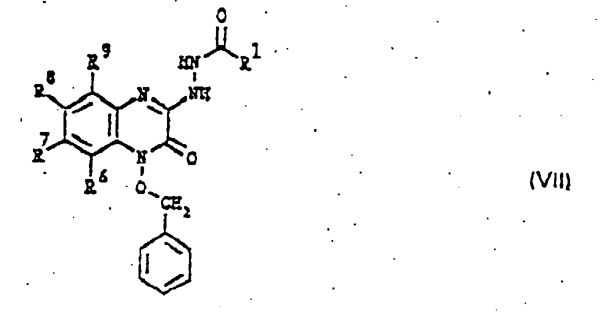 worin R¹, R⁶, R⁷, R⁸ und R⁹ die oben angegebenen Bedeutungen haben, acyliert, die Verbindung zu einer Verbindung der Formel VIII 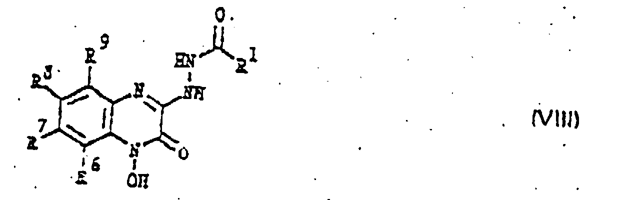 worin R¹, R⁶, R⁷, R⁸ und R⁹ die oben angegebenen Bedeutungen haben, hydrogenolytisch spaltet und durch thermische Cyclisierung und gleichzeitige Desoxygenierung eine Verbindung nach Anspruch 1 herstellt, oder
b) eine Verbindung der Formel IX 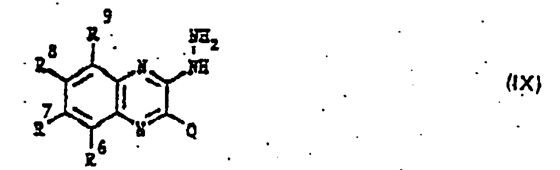 worin R⁶, R⁷, R⁸ und R⁹ die oben angegebenen Bedeutungen haben und Q für Br, Cl oder I steht, mit einer Verbindung der allgemeinen Formel VI
R¹-COCl (VI)
worin R¹ die oben angegebene Bedeutung hat, zu einer Verbindung der Formel XI 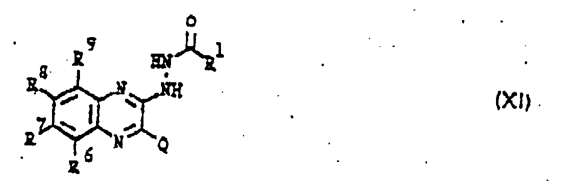 worin R¹, R⁶, R⁷, R⁸ und R⁹ die oben angegebenen Bedeutungen haben und Q für Br, Cl oder I steht, umsetzt und dann durch Cyclisierung und anschließende Hydrolyse oder durch gleichzeitige Cyclisierung und Hydrolyse eine Verbindung nach Anspruch 1 herstellt, oder
c) eine Verbindung der Formel XII 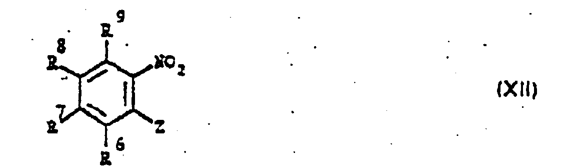 worin R⁶, R⁷, R⁸ und R⁹ die oben angegebenen Bedeutungen haben und Z entweder für Halogen oder für C₁₋₆-Alkoxy steht, mit mono-, di- oder trimethoxysubstituiertem Benzylamin zu einer Verbindung der Formel XIII 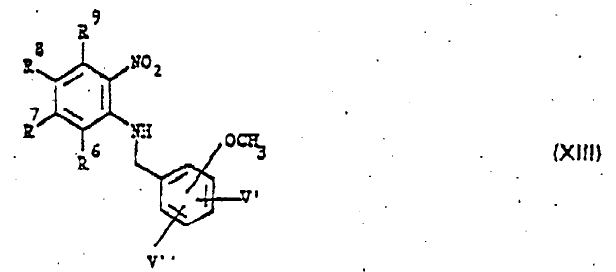 worin R⁶, R⁷, R⁸ und R⁹ die oben angegebenen Bedeutungen haben und V' und V" unabhängig voneinander für Wasserstoff oder Methoxy stehen, substituiert, die Verbindung mit Ethyloxalylchlorid zu einer Verbindung der Formel XIV 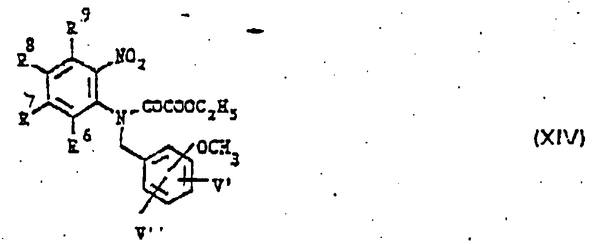 worin R⁶, R⁷, R⁸ und R⁹ die oben angegebenen Bedeutungen haben und V' und V" unabhängig voneinander für Wasserstoff oder Methoxy stehen, umsetzt, dann entweder durch Hydrierung zur intermediären cyclisierten N-Hydroxyverbindung und anschließende Desoxygenierung oder durch cyclisierende Hydrierung eine Verbindung der Formel XV 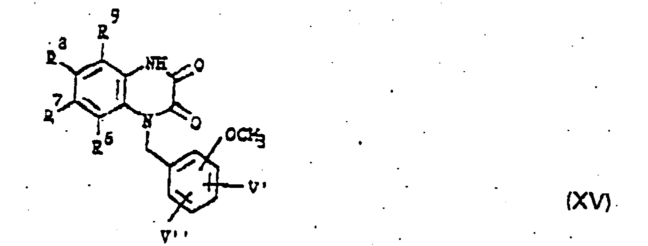 worin R⁶, R⁷, R⁸ und R⁹ die oben angegebenen Bedeutungen haben und V' und V" unabhängig voneinander für Wasserstoff oder Methoxy stehen, herstellt, die Verbindung der Formel XV halogeniert, die erhaltene Verbindung mit Hydrazin umsetzt, dann mit einem Acylchlorid der oben definierten allgemeinen Formel VI acyliert, danach zu einer Verbindung der Formel XVI 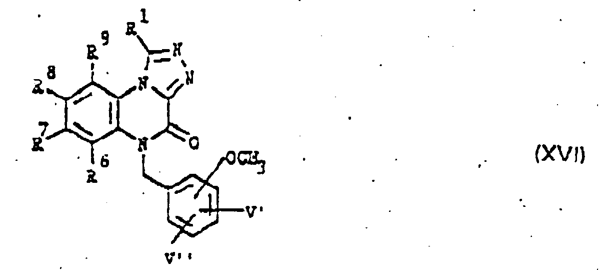 worin R¹, R⁶, R⁷, R⁸ und R⁹ die oben angegebenen Bedeutungen haben und V' und V" unabhängig voneinander für Wasserstoff oder Methoxy stehen, cyclisiert und zu einer Verbindung nach Anspruch 1 hydrolysiert, oder
d) eine Verbindung nach Anspruch 1, mit wäßriger Base zu einer Verbindung nach Anspruch 1 hydrolysiert, oder
e) eine Verbindung nach Anspruch 1 mit Halogentrimethylsilan zu einer Verbindung nach Anspruch 1 umsetzt.

10. Verfahren zur Herstellung einer pharmazeutischen Zusammensetzung zur Verwendung bei der Behandlung einer mit Hyperaktivität der exzitatorischen Neurotransmitter verbundenen Indikation, bei dem man eine Verbindung nach Anspruch 1 oder ein pharmazeutisch unbedenkliches Salz davon in eine galenische Dosisform bringt.

## Revendications

1. Composé qui est
la 1-(éthoxyhydroxyphosphorylméthyl)-8-(1H-imidazol-1-yl)-7-trifluorométhyl[1,2,4]triazolo[4,3-a]quinoxalin-4(5H)-one ;
la 8-(1H-imidazol-1-yl)-1-phosphonométhyl-7-trifluorométhyl[1,2,4]triazolo[4,3-a]quinoxalin-4(5H)-one;
la 1-(éthoxyhydroxyphosphorylméthyl)-8-(2-isopropyl-lH-imidazol-1-yl)-7-trifluorométhyl[1,2,4]triazolo[4,3-a]-quinoxalin-4(5H)-one ;
la 8-(2-isopropyl-1H-imidazol-1-yl)-1-phosphonométhyl-7-trifluorométhyl[1,2,4]triazolo[4,3-a]quinoxalin-4(5H)-one ;
et sels pharmaceutiquement acceptables de celui-ci.

2. Composition pharmaceutique comprenant, en tant que composant actif, un composé selon la revendication 1 ou un sel pharmaceutiquement acceptable de celui-ci, et un support ou diluant pharmaceutiquement acceptable.

3. Composition pharmaceutique selon la revendication 2, sous la forme d'une unité de dosage contenant environ 10-200 mg du composé actif.

4. Utilisation d'un composé selon la revendication 1 ou d'un sel pharmaceutiquement acceptable de celui-ci en tant que médicament.

5. Utilisation d'un composé selon la revendication 1 ou d'un sel pharmaceutiquement acceptable de celui-ci pour la préparation d'un médicament destiné à traiter une indication liée à l'hyperactivité de neurotransmetteurs excitateurs.

6. Composition pharmaceutique appropriée pour une utilisation dans le traitement d'une indication liée à l'hyperactivité des neurotransmetteurs excitateurs, qui comprend, en tant que composant actif, un composé selon la revendication 1 ou un sel pharmaceutiquement acceptable de celui-ci, et un support ou diluant pharmaceutiquement acceptable.

7. Utilisation d'un composé selon la revendication 1 pour le traitement d'une indication liée à l'hyperactivité des neurotransmetteurs excitateurs chez un sujet en ayant besoin, qui comprend l'étape d'administration audit sujet d'une quantité antagoniste de l'AMPA et neurologiquement efficace d'un composé selon la revendication 1 ou d'un sel pharmaceutiquement acceptable de celui-ci.

8. Utilisation selon la revendication 7, dans laquelle l'indication est liée à l'ischémie cérébrale.

9. Méthode de préparation des composés selon la revendication 1 qui comprend,
a) l'alkylation d'un composé de formule II 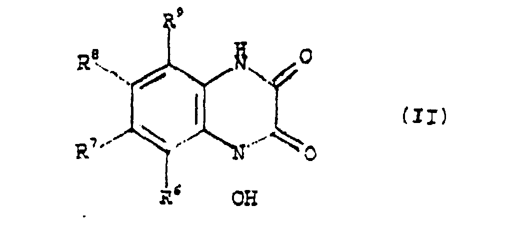 dans laquelle R⁶ et R⁹ sont un hydrogène, R⁷ est Cl₃ et R⁸ est un imidazolyle ou un isopropylimidazolyle, avec un halogénure de benzyle pour former un composé de formule III 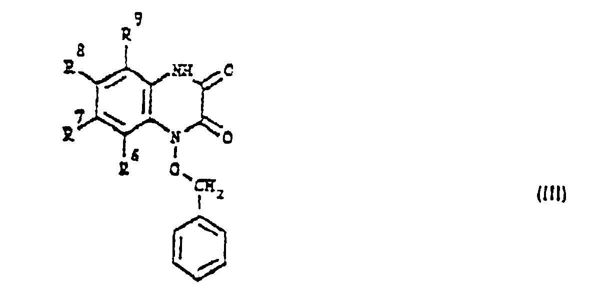 dans laquelle R⁶, R⁷, R⁸ et R⁹ ont les significations définies ci-dessus, et l'halogénation du composé pour former un composé de formule IV 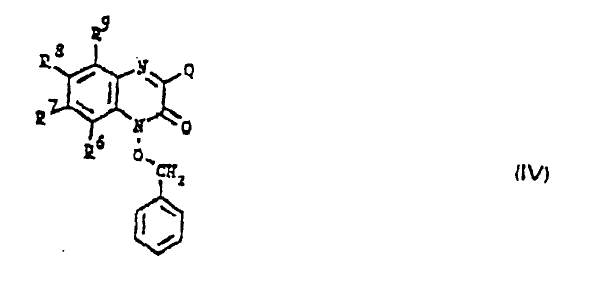 dans laquelle R⁶, R⁷, R⁸ et R⁹ ont les significations définies ci-dessus et Q est Br, Cl ou I ; et la réaction du composé avec de l'hydrazine pour former un composé de formule V 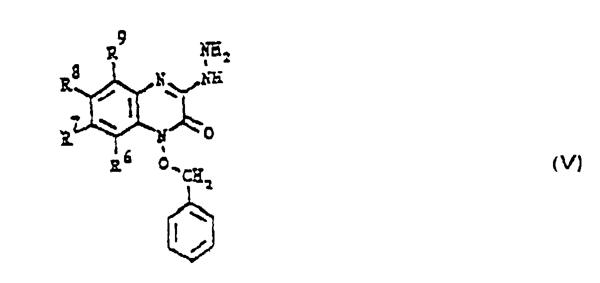 dans laquelle R⁶, R⁷, R⁸ et R⁹ ont les significations définies ci-dessus, et l'acylation du composé avec un chlorure d'acyle de formule générale VI
R¹-COCl (VI)
dans laquelle R¹ est un éthoxyhydroxyphosphorylméthyle ou un phosphonométhyle, pour former un composé de formule VII 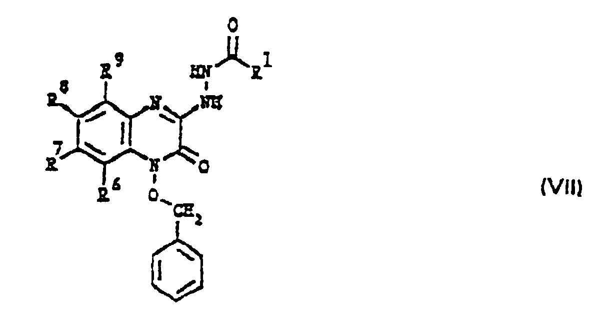 dans laquelle R¹, R⁶, R⁷, R⁸ et R⁹ ont les significations définies ci-dessus, et l'hydrogénolyse du composé pour former un composé de formule VIII 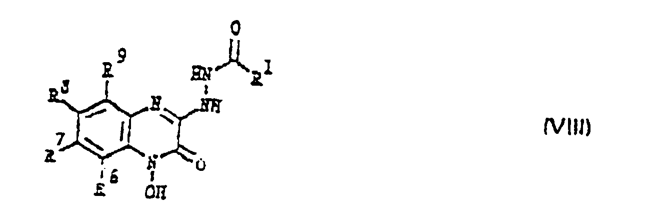 dans laquelle R¹, R⁶, R⁷, R⁸ et R⁹ ont les significations définies ci-dessus, et suivie par une cyclisation thermique et une désoxygénation simultanée pour former un composé selon la revendication 1, ou
b) la réaction d'un composé de formule IX 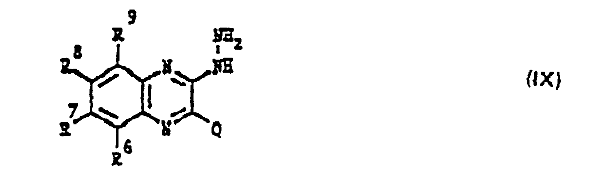 dans laquelle R⁶, R⁷, R⁸ et R⁹ ont les significations définies ci-dessus, et Q est Br, Cl ou I, avec un composé de formule générale VI
R¹-COCl (VI)
dans laquelle R¹ a la signification telle que définie ci-dessus, pour former un composé de formule XI 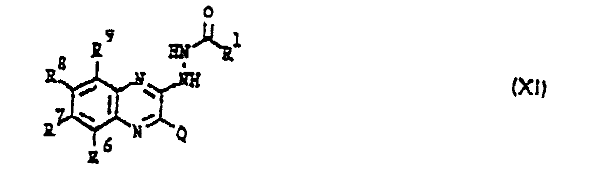 dans laquelle R¹, R⁶, R⁷, R⁸ et R⁹ ont les significations définies ci-dessus et Q est Br, Cl ou I, et puis soit une cyclisation suivie d'une hydrolyse soit une cyclisation et une hydrolyse simultanées pour former un composé selon la revendication 1, ou
c) la substitution d'un composé de formule XII 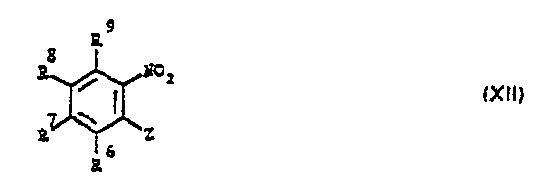 dans laquelle R⁶, R⁷, R⁸ et R⁹ ont les significations définies ci-dessus, et Z est soit un halogène soit un alcoxy en C₁₋₆, avec une benzylamine mono-, di- ou triméthoxy-substituée pour former un composé de formule XIII 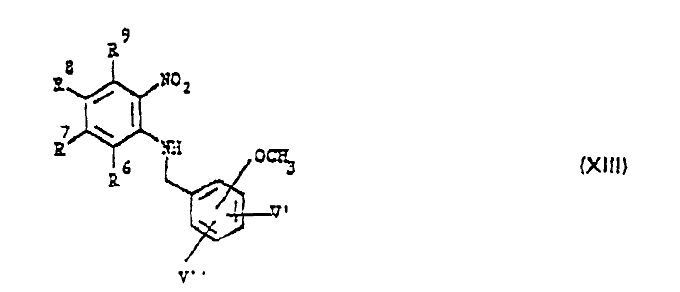 dans laquelle R⁶, R⁷, R⁸ et R⁹ ont les significations définies ci-dessus, et V' et V" sont indépendamment un hydrogène ou un méthoxy et la réaction du composé avec du chlorure d'éthyloxalyle pour former un composé de formule XIV 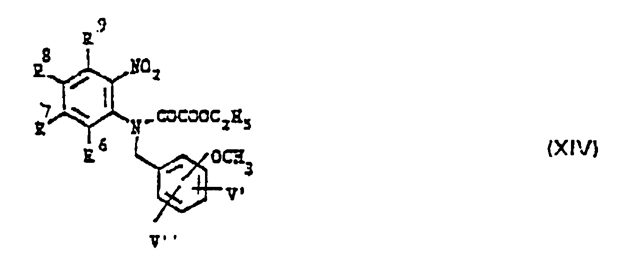 dans laquelle R⁶, R⁷, R⁸ et R⁹ ont les significations définies ci-dessus, et V' et V" sont indépendamment un hydrogène ou un méthoxy, et puis soit une hydrogénation pour former le composé N-hydroxylé cyclisé intermédiaire suivie d'une désoxygénation soit une cyclisation par hydrogénation pour former un composé de formule XV 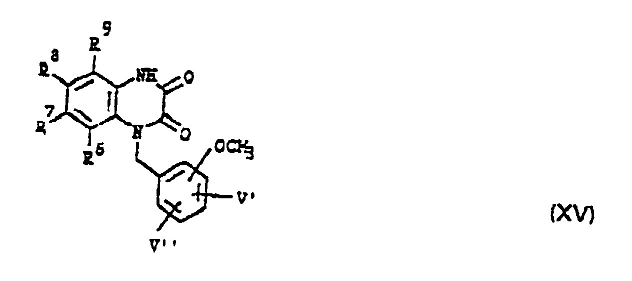 dans laquelle R⁶, R⁷, R⁸ et R⁹ ont les significations définies ci-dessus, et V' et V" sont indépendamment un hydrogène ou un méthoxy, l'halogénation du composé de formule XV, la réaction du composé résultant avec de l'hydrazine suivie d'une acylation avec un chlorure d'acyle de formule générale VI, comme défini ci-dessus, et puis une cyclisation pour former un composé de formule XVI 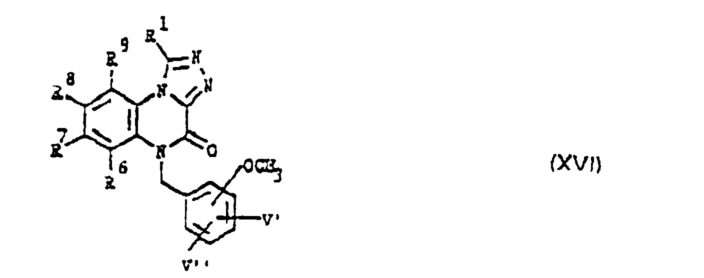 dans laquelle R¹, R⁶, R⁷, R⁸ et R⁹ ont les significations définies ci-dessus, et V' et V" sont indépendamment un hydrogène ou un méthoxy, et l'hydrolyse pour former un composé selon la revendication 1, ou
d) l'hydrolyse d'un composé selon la revendication 1 avec une base aqueuse pour former un composé selon la revendication 1, ou
e) la réaction d'un composé selon la revendication 1 avec un halogénotriméthylsilane pour former un composé selon la revendication 1.

10. Procédé de préparation d'une composition pharmaceutique destinée à être utilisée dans le traitement d'une indication liée à l'hyperactivité des neurotransmetteurs excitateurs, lequel procédé comprend la mise d'un composé selon la revendication 1 ou d'un sel pharmaceutiquement acceptable de celui-ci sous une forme de dosage galénique.
